# EUROPEAN PATENT APPLICATION

(11) **EP 2 481 403 A1**
(43) Date of publication of application: **01.08.2012**
(21) Application number: 11152896.4
(22) Date of filing: 01.02.2011
(51) Int. Cl.: A61K 31/215, A61K 31/245, A61P 1/06, A61K 9/16

(54) **Pharmaceutical combination of otilonium and trimebutine**

(71) Applicant: Deva Holding Anonim Sirketi, 34303 Istanbul (TR)
(72) Inventor: Haas, Philipp Daniel, 34303, ISTANBUL (TR); Sivasligil, Ramazan, 34303, ISTANBUL (TR); Koc, Fikret, 34303, ISTANBUL (TR); Kandemir, Levent, 34303, ISTANBUL (TR)

(57) **Abstract**

This invention is related to pharmaceutical combination of otilonium or pharmaceutically acceptable salts thereof and trimebutine or pharmaceutically acceptable salts thereof.

## Description

### Technical Field

This invention is related to pharmaceutical combination of otilonium or pharmaceutically acceptable salts thereof and trimebutine or pharmaceutically acceptable salts thereof.

### Background Art

EP 0270503 B (A MENARINI INDUSTRIE FARMACEUTICHE RIUNITE S.R.L) 08.06.1988 discloses a pharmaceutical composition of otilonium for topical use in the gastrointestinal tract.

### Summary of invention

The invention relates to a combination of

(i) otilonium or a pharmaceutically acceptable salt thereof and

(ii) trimebutine or a pharmaceutically acceptable salt thereof and

(iii) preferably an excipient or mixtures of excipients.

Thus combination of trimebutine which is an opioid receptor agonist and otilonium which is a muscarinic receptor antagonist effectively treat and/or relief symptoms of IBS and related illnesses.

### Technical Problem

Trimebutine maleate is a noncompetitive spasmolytic agent. It possesses moderate opiate receptor affinity.

Trimebutine is an opioid receptor agonist which has gastrointestinal motility regulator and antispasmodic properties

Otilonium bromide is a muscarinic receptor antagonist, which has spasmolytic action on the smooth muscle of the digestive tract. The action mechanism of otilonium bromide consists in the blockade of the calcium channels present in the smooth muscle.

Both otilonium and trimebutine are essentially used in the treatment of irritable bowel syndrome.

In practice, medical doctors prescribe to patients who suffer from irritable bowel syndrome either trimebutine or otilonium and they expect panacea from trimebutine or otilonium. But if irritable bowel syndrome arises due to different mechanisms of both muscarinic receptors and opioid receptors at the same time, otilonium or trimebutine alone does not effect on disease.

An important percentage of patients who are suffering from IBS are experiencing swallowing disorder as well. Dysphagia patients are unable to perform the necessary reflex or may be feeling sick or unwilling and/or unable to swallow tablets. This is especially the case of pharmaceuticals for paediatric or geriatric use.

### Solution to Problem

In accordance with this invention irritable bowel syndrome is treated with pharmaceutically effective amount of trimebutine and otilonium in a combination or in a kit. This solution provides that a fixed dosage form or a kit covers both opioid receptor agonist and muscarinic receptor antagonist by means of trimebutine and otilonium respectively. It means that fixed dosage form or kit eliminate problems arising from both opioid receptor and muscarinic receptor.

In order to enhance the patient compliance in the treatment of irritable bowel syndrome, the compositions of this invention are also presented for patients who suffer from dysphagia. For patients who are experiencing dysphagia, presented formulations are beneficial. Patients benefit from swallowing only one newly developed combination tablet of otilonium and trimebutine instead of two different tablets of otilonium and trimebutine in two times.

### Description of embodiments

Otilonium, a quaternary ammonium derivative, is clinically used for the treatment of hypermotility and hypersensitivity disorders of the intestine in particular it is indicated and marketed in many countries for the treatment of irritable bowel syndrome.

Trimebutine (a peripheral opiate receptor antagonist) is used in treatment of gastrointestinal disorders related to smooth muscle spasm.

This invention is directed to

(i) otilonium or a pharmaceutically acceptable salt thereof and

(ii) trimebutine or a pharmaceutically acceptable salt thereof and

(iii) optionally an excipient or mixture of excipients.

In this invention the term of "otilonium" and the term of "trimebutine" cover pharmaceutically acceptable bases, salts, isomers, racemates, racemic mixtures, individual diasteriomers, enantiomers. Furthermore, some of the crystalline forms for compounds of the present invention may exist as polymorphs and as such are intended to be included in the present invention. In addition, some of the compounds of the invention may form solvates with water or common organic solvents. Such solvates and hydrates, as well as anhydrous compositions, are encompassed within the scope of this invention.

The term "composition" or "combination" covers each other and can be used instead of other one.

All forms and mixtures thereof are included within the scope of this invention.

According to preferred embodiment of the invention is:

(i) otilonium or a pharmaceutically acceptable salt thereof and

(ii) trimebutine or a pharmaceutically acceptable salt thereof and

(iii) optionally an excipient or mixture of excipients for the manufacture of a medicament for the treatment or prevention of:
- Gastro intestinal symptoms due to chronic gastritis
- Gastro intestinal tract spasm
- Irritable bowel syndrome and/or symptoms associated with the irritable bowel syndrome
- Abdominal distention, abdominal pain, nausea, constipation, diarrhea, eructation
- In postoperative paralytic ileus in order to accelerate the resumption of the intestinal transit following abdominal surgery

In another aspect this invention, otilonium is present in an amount of from 10 mg to 150 mg, preferably in an amount of from 10 mg to 60 mg and most preferably 40 mg and trimebutine is present in an amount of from 10 mg to 500 mg, preferably in an amount of from 20 mg to 400 mg and most preferably 200 mg.

Preferably combination is administered three times daily.

Preferred salt of trimebutine is maleate and preferred salt of otilonium is bromide.

The pharmaceutical compositions according to the invention can be prepared for enteral, such as oral or rectal, and parenteral administration to mammals (warm-blooded animals), including human being.

For the manufacture of solid dosage forms, for example, the active ingredients may be mixed with the excipients and/or additives and granulated in a wet or dry process. Direct compression, melt granulation, melt congealing, extrusion process and such processes can be applied. Granules or powder can be filled directly into capsules or compressed into tablet cores.

These are prepared in a manner for example by means of mixing, granulating, dissolving, lyophilizing processes.

The pharmaceutical preparations contain, for example, from about 10 % to about 90 % of the active ingredients.

The components of a pharmaceutical combination can be dosed independently or by use of different fixed combinations. In other words administration may take place in the form of one fixed combination with two or more active ingredients, or by simultaneously administering two or more active ingredients that are formulated independently.

As a yet another aspect, this invention encompasses a kit or commercial package comprising a plurality of separate containers, wherein at least one container contains combination of otilonium and trimebutine or a pharmaceutically acceptable salts thereof and at least one different container contains otilonium or trimebutine or pharmaceutically acceptable salts thereof.

In accordance with this invention the kit comprising (i) therapeutically effective amount otilonium and optionally an acceptable carrier or diluent in a unit dosage form and (ii) therapeutically effective amount of trimebutine and an acceptable carrier or diluent in a unit dosage form and (iii) a container.

According to this invention, each unit dosage form is discrete dosage forms that are packaged together. The kits of the invention can include container as separate compositions such as a divided bottle or a divided packet. Separate unit dosage forms can also be contained in undivided container. The pharmaceutical kits can include instructions as a separate sheet or optionally printed on the containers.

The pharmaceutical combination can concurrently, separately or sequentially be administered with an instruction.

It is preferred that otilonium and trimebutine be co-administered concurrently on three times-per-day dosing schedule. However, varying dosing schedules, such as once per day (o.d), or more times per day such as b.i.d or t.i.d, are also encompassed herein.

A single oral dosage formulation comprised of both otilonium and trimebutine is preferred. The single dosage formulation will provide convenience for the patient, where patients who needs multiple medications.

For the present invention in combinations, otilonium : trimebutine ratio range is selected from 1:90 to 90:1 by weight. More preferably range is selected from 1:60 to 60:1 and most preferably range is selected from1:10 to 10:1.

Total % quantity of drug substances, otilonium bromide and trimebutine maleate, may vary between 10-90% of total weight of pharmaceutical dosage form.

Preferably, total % quantity of drug substances varies between 20 - 80% of total weight of pharmaceutical dosage form.

Total % quantity of diluent(s) may vary between 1 - 60% of total weight of pharmaceutical dosage form.

Preferably, total % quantity of diluent(s) varies between 2 - 50% of total weight of pharmaceutical dosage form.

Total % quantity of binder(s) may vary between 5 - 15% of total weight of pharmaceutical dosage form.

Preferably, total % quantity of binder(s) varies between 6 - 12% of total weight of pharmaceutical dosage form.

Total % quantity of disintegrant(s) may vary between 2 - 15% of total weight of pharmaceutical dosage form.

Preferably, total % quantity of disintegrant(s) varies between 3 - 12% of total weight of pharmaceutical dosage form.

Total % quantity of lubricant(s) may vary between 0.1 - 2% of total weight of pharmaceutical dosage form.

Preferably, total % quantity of lubricant(s) varies between 0.3 - 1.5% of total weight of pharmaceutical dosage form.

Pharmaceutical combination may be prepared using methods as below without limiting scope of this invention;
- Both active agents are separately granulated and then granulates are combined or,
- One active agent is granulated and then granulate is combined with non granulated active agent or,
- Both active pharmaceutical ingredients are granulated together or,
- One active agent is dissolved alone or with one or more excipients and then other active agent alone or with one or more excipients are coated with said solution
- One active agent is dispersed alone or with one or more excipient and then other active agent alone or with one or more excipients are coated or adhered with said dispersion

Said solution or dispersion, mention in previous paragraph comprises;

(i) an inorganic acid or mixtures of inorganic acids or,

(ii) an organic acid or mixtures of organic acids or,

(iii) mixtures of organic acid or organic acids and inorganic acid or inorganic acids and

(v) an organic solvent or mixtures of organic solvents and

(vi) optionally de-ionized water and,

(vii) optionally binder or binders.

Organic solvents used in preparation of solution or dispersion are but not limited to, alcohols such as methanol, ethanol, propyl alcohol, isopropyl alcohol, 2-methyl-1-propanol etc. ; ketones such as acetone, methyl ethyl ketone, methyl isobutyl ketone etc. ; esters such as methyl acetate, ethyl acetate, propyl acetate, isopropyl acetate, ethyl formate etc. ; hydrocarbons such as heptane ; halogenated hydrocarbons such as dichloromethane.

Organic acids are but not limited to formic acid, acetic acid, fumaric acid, tartaric acid, maleic acid, citric acid, succinic acid, malic acid, methanesulfonic acid,p-toluenesulfonic acid.

Inorganic acid is hydrochloric acid in the form of solution in water.

A further object of the instant invention involves following preparations :
- Otilonium and trimebutine are separately granulated and then they are separately compressed into mini tablets and filled into capsule altogether or
- Otilonium and trimebutine are separately granulated and then they are separately compressed into tablet and filled into capsule or
- One of the active agents, otilonium or trimebutine, is granulated and compressed into tablet or mini-tablets and then combined with granules of other active agent, otilonium or trimebutine, into capsule or
- Otilonium and trimebutine are separately granulated and then directly filled into capsule without tabletting or
- One of the active agents, otilonium or trimebutine, is prepared through direct compression into tablet or mini-tablets and then combined with granules of other active agent, otilonium or trimebutine, into capsule.

Pharmaceutical preparations according to the invention are, for example, tablet, bilayer tablet, tablet in tablet, sugar coated tablet, capsule, tablet in capsule, inlay tablet, pellets in capsule, suppositories, powders, granules, granules in capsule, effervescent tablet, readily dissolved granule, effervescent granules, effervescent pellets, effervescent dry powder, readily dispersible granule and other dosage forms suitable for oral administration. Liquid preparations may be such as solutions, ampoules, elixir, intravenous and intramuscular forms, suspensions or emulsions.

In an embodiment, the present invention provides tablet-in-tablet compositions comprising: a) a core tablet comprising: trimebutine or pharmaceutically acceptable salts thereof and a pharmaceutically acceptable carrier or carriers b) a compressed outer tablet layer comprises : otilonium or pharmaceutically acceptable salts thereof and a pharmaceutically acceptable carrier or carriers. Alternatively, tablet-in-tablet compositions comprising: a) a core tablet comprising: otilonium or pharmaceutically acceptable salts thereof and a pharmaceutically acceptable carrier or carriers b) a compressed outer tablet layer comprising: trimebutine or pharmaceutically acceptable salts thereof and a pharmaceutically acceptable carrier or carriers.

In another embodiment, this invention embraces bilayer tablet. Bilayer tablet comprises; a) a first layer containing trimebutine or pharmaceutically acceptable salts thereof and a pharmaceutically acceptable carrier or carriers and b) a second layer containing otilonium or pharmaceutically acceptable salts thereof and a pharmaceutically acceptable carrier or carriers. Alternatively, bilayer tablet comprises a) a first layer containing otilonium or pharmaceutically acceptable salts thereof and a pharmaceutically acceptable carrier or carriers and b) a second layer containing trimebutine or pharmaceutically acceptable salts thereof and a pharmaceutically acceptable carrier or carriers.

The expression bilayer is not limited to two layers also multilayer tablets are meant with this expression.

The first and the second layers of bilayer tablet can also be arranged side-by-side.

In still another embodiment, this invention delineates an inlay tablet. The inlay tablet comprises that inner tablet is encompassed by an outer tablet wherein at least a portion of one surface of the inner tablet is not surrounded by outer tablet. The inner tablet comprises otilonium and the outer tablet comprises trimebutine or vice versa. An excipient or mixture of excipients can be used.

In still another aspect, this invention embraces capsule in capsule formulations wherein smaller size capsule is encapsulated into a larger capsule. Capsule-in-capsule consists of an external capsule and internal capsule (inner capsule) located therein. It is preferred that smaller size capsule is filled with otilonium and optionally excipients and larger capsule is filled with trimebutine and optionally excipients. Capsule in capsule operations can be performed via Modu-C (HarroHofliger).

Another aspect of this invention is to provide spray dried powders of trimebutine or otilonium or mixtures of otilonium and trimebutine. To prepare slurry or solution for spray drying, suitable excipients such as binders and disintegrants may be combined with the active agents. The slurry or solution of active agents can be directly spray dried or they can be combined with one or more excipients before spray drying. The slurry or solution can be spray dried by the use of conventional spray drying apparatuses like vertical spray dryer or fluid spray dryer. For instance Buchi Mini Spray Dryer B-290 can be used.

In still another embodiment of this invention is directed to effervescent tablet, effervescent granules, effervescent pellets and effervescent dry powder. Effervescent forms use of a chemical reaction between a soluble acid and alkali metal carbonate with the help of water to make CO₂ gas, which can give a fizzy taste in the mouth. Any conventional effervescent agent, for example a mixture of a substance which on dissolution in water forms an acid reacting solution, and a pharmaceutically acceptable carbonate can be used as effervescent agent. The acid component of the composition can be any suitable acid for effervescent compositions. Typically, the acid is organic or mineral acid that is safe and approved for pharmaceutical and/or nutritional purposes and which provides effective and rapid effervescent disintegration upon contact with water and the alkaline effervescent compound. The acid may be selected from food acids, acid anhydrides and acid salts.

As a yet further aspect, prepared granulates can be filled into capsules. Capsules can be consisted of separated compartments and each active ingredient and it's granulates can be filled into each compartment.

According to this invention, combination includes one or more pharmaceutically acceptable excipients, carriers, or diluents. In formulations, surfactants, diluents, sweeteners, disintegrants, direct compression agents, acids for effervescent forms, binders, lubricants, glidants, colorants, alkaline effervescing agents, flavors and mixtures thereof can be used.

Diluents are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharmaburst™, mixtures thereof and the like.

Glidants are, but not limited to, silicon dioxide, colloidal silicon dioxide, calcium silicate, magnesium silicate, magnesium trisilicate, talc, starch, mixtures thereof and the like.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Lubricants are, but not limited to, calcium stearate, glyceryl monostearate, glyceryl behenate, glyceryl palmitostearate, hydrogenated vegetable oil, light mineral oil, magnesium stearate, mineral oil, polyethylene glycol, poloxamer, sodium benzoate, sodium lauryl sulfate, sodium stearyl fumarate, stearic acid, talc, zinc stearate, mixtures thereof and the like .

Disintegrants are, but not limited to, sodium starch glycolate, sodium carboxymethyl cellulose, calcium carboxymethyl cellulose, croscarmellose sodium, crospovidone, alginic acid, chitosan, methyl cellulose, microcrystalline cellulose, powdered cellulose,lower alkylsubstituted hydroxypropyl cellulose, polacrilin potassium, starch, pregelatinized starch, sodium alginate, mixtures thereof or whatsoever.

Direct compression agents are, but not limited to, pregelatinized starch, polyvinylpyrrolidone, methylcellulose, microcrystalline cellulose, sucrose, lactose, dextrose, sorbitol, mannitol, lactitol,xylitol, modified calcium salt, granulated corn starch, modified rice starch, compressible sugars such as Destab™, dextrates such as Emdex ® dicalcium phosphate, hydroxypropylcellulose , methylcellulose , hydroxypropylmethylcellulose, polyethylene glycol, amylose,anhydrous calcium hydrogen phosphate, calcium sulphate, tribasic calcium phosphate, dibasic calcium phosphate, low-crystallinity powdered cellulose,silicified microcrystalline cellulose,chitin,chitosan hydrochloride,copovidone,croscarmellose sodium,dextrose, anhydrous lactose,anhydrous alpha lactose,anhydrous beta lactose,agglomerated lactose, spray-dried lactose, maltodextrin, mixtures thereof and the like.

Acids are, but not limited to, citric acid, tartaric acid, ascorbic acid, fumaric acid, malic acid, adipic and succinic acid, acetyl salicylic acid, nicotinic acid, citric anhydride, succinic anhyride, glutamic anhydride, sodium hydrogen phosphate, disodium dihydrogen pyrophospate, acid citrate salts, aminoacid hydrochlorides, mixtures thereof and the like.

Alkaline effervescing agents are, but not limited to, sodium bicarbonate, sodium carbonate, potassium bicarbonate and potassium carbonate, sodium sesquicarbonate, sodium glycine carbonate, L-lysine carbonate, arginine carbonate, amorphous calcium carbonate, mixtures thereof and the like.

Flavors are, but not limited to, cinnamon oil,essence of apple, essence of pear, essence of peach, essence of grape, essence of strawberry, essence of raspberry, essence of cherry, essence of plum, essence of pineapple, essence of apricot, oil of wintergreen, peppermint oils, clove oil, bay oil, anise oil, eucalyptus, thyme oil, cedar leaf oil, oil of nutmeg, oil of sage, oil of bitter almonds, cassia oil, citrus oils such as lemon, orange, grape, lime and grapefruit , vanilla, benzaldehyde , aldehyde C-8 , aldehyde C-9, aldehyde C-12 , tolyl aldehyde, mixtures thereof and the like.

Sweeteners are but not limited to, corn syrup, dextrose, cyclamate, invert sugar, fructose, saccharin, aspartame, acesulfame-K, Stevia rebaudiana, sucralose, sorbitol, mannitol, xylitol, mixtures thereof and the like.

### Example 1 (Unit Formula: Single Tablet)

Otilonium Bromide + Trimebutine Maleate Single Tablet:

**Table 1**

| | **mg/tablet** | **% (w/w)** | **Function** |
|---|---|---|---|
| **Active Substances** | | | |
| Otilonium Bromide | 40.00 | 8.00 | Drug Substance |
| Trimebutine Maleate | 200.00 | 40.00 | Drug Substance |

| **Excipients** | | | |
|---|---|---|---|
| Lactose Monohydrate | 110.00 | 22.00 | Diluent |
| Pregelatinized Starch | 35.00 | 7.00 | Binder |
| Sodium Starch Glycolate | 50.00 | 10.00 | Disintegrant |
| Microcrystalline Cellulose (Avicel PH 102) | 60.00 | 12.00 | Diluent / Compression Aid |
| Magnesium Stearate | 5.00 | 1.00 | Lubricant |
| Total Core Tablet Weight | 500.00 | 100.00 | |

Core tablets are optionally coated with dispersion of film coating material (HPMC or PVA based) either dispersed in water or water and alcohol mixture or organic solvent. Total film coating applied may vary 1 - 5 % (w/w) of core tablet. Preferred applied film coating is 3 % (w/w) of core tablet.

### Example 2 (Manufacturing Process of Example 1)

Manufacturing Process of Example 1 :
1. Lactose Monohydrate, ½ of Sodium Starch Glycolate and Trimebutine Maleate are mixed.
2. Otilonium Bromide is dissolved in water. Pregelatinized Starch is dispersed in this solution.
3. Powder mixture in Step A is granulated with dispersion in Step B using high shear mixer, Collette Gral Granulator.
4. Wet granules are dried using Fluid Bed Drier, Aeromatic T7, until moisture content is less than 4%.
5. Dry granules are mixed with ½ of Sodium Starch Glycolate and Avicel PH 102.
6. Magnesium Stearate is added and mixed.
7. Core tablets are compressed with suitable punches having average target weight 500 mg/tablet.
8. Core tablets are optionally film coated. Opadry II 85F18422 White can be used as film coating agent.
9. 16% (w/w) dispersion of Opadry II 85F18422 White is prepared in water.
10. Core tablets are film coated with above dispersion using Vector Hi-Coater Film Coating Machine until desired film coating applied.

### Example 3 (Unit Formula: Single Tablet)

Otilonium Bromide + Trimebutine Maleate Single Tablet:

**Table 2**

| | **mg/tablet** | **% (w/w)** | **Function** |
|---|---|---|---|
| **Active Substances** | | | |
| Otilonium Bromide | 40.00 | 8.00 | Drug Substance |
| Trimebutine Maleate | 200.00 | 40.00 | Drug Substance |

| **Excipients** | | | |
|---|---|---|---|
| Lactose Monohydrate | 85.00 | 17.00 | Diluent |
| Pregelatinized Starch | 35.00 | 7.00 | Binder |
| Sodium Starch Glycolate | 50.00 | 10.00 | Disintegrant |
| Microcrystalline Cellulose (Avicel PH 102) | 85.00 | 17.00 | Diluent / Compression Aid |
| Magnesium Stearate | 5.00 | 1.00 | Lubricant |
| Total Core Tablet Weight | 500.00 | 100.00 | |

Core tablets are optionally coated with dispersion of film coating material (HPMC or PVA based) either dispersed in water or water and alcohol mixture or organic solvent. Total film coating applied may vary 1 - 5 % (w/w) of core tablet. Preferred applied film coating is 3 % (w/w) of core tablet.

### Example 4 (Manufacturing Process of Example 3)

Manufacturing Process of Example 3 by Spray Drying:
1. Lactose Monohydrate, Otilonium Bromide, 1/3 of Sodium Starch Glycolate, Pregelatinized Starch and Trimebutine Maleate are mixed in Fluid Bed Granulator (Vector VFC-30X).
2. Powder mixture in Step A is granulated with water and dried simultaneously.
3. Drying is continued until moisture content is less than 4%.
4. Dry granules are mixed with remaining Sodium Starch Glycolate and Avicel PH 102.
5. Magnesium Stearate is added and mixed.
6. Core tablets are compressed with suitable punches having average target weight 500 mg/tablet.
7. Core tablets are optionally film coated. Opadry YS 1 7002 White can be used as film coating agent.
8. 12% (w/w) dispersion of Opadry YS 1 7002 White is prepared in water+alcohol (60:40) mixture.
9. Core tablets are film coated with above dispersion using perforated coating machine, GS Perfima, until desired film coating applied.

### Example 5 (Tablet-in-tablet: Unit Formula)

Otilonium Bromide + Trimebutine Maleate Tablet in Tablet:

**Table 3**

| | **mg/tablet** | **% (w/w)*** | **Function** |
|---|---|---|---|
| Otilonium Tablet | | | |
| Otilonium Bromide | 40.00 | 8.00 | Drug Substance |
| Lactose Monohydrate | 31.00 | 6.20 | Diluent |
| Pregelatinized Starch | 12.00 | 2.40 | Dry Binder |
| Crospovidone | 6.00 | 1.20 | Disintegrant |
| Magnesium Stearate | 1.00 | 0.20 | Lubricant |
| Total Otilonium Tablet Weight | 90.00 | | |

| Trimebutine Granule | | | |
|---|---|---|---|
| Trimebutine Maleate | 200.00 | 40.00 | Drug Substance |
| Lactose Monohydrate | 85.00 | 17.00 | Diluent |
| PVP K-30 | 32.00 | 6.40 | Binder |
| Croscarmellose Sodium | 40.00 | 8.00 | Disintegrant |
| Microcrystalline Cellulose (Avicel PH 102) | 50.00 | 10.00 | Diluent / Compression Aid |
| Magnesium Stearate | 3.00 | 0.60 | Lubricant |
| Total Trimebutine Granule Weight | 410.00 | | |
| Total Core Tablet Weight | 500.00 | | |

* % values are calculated according to final core tablet weight.

Core tablets are optionally coated with dispersion of film coating material (HPMC or PVA based) either dispersed in water or water and alcohol mixture or organic solvent. Total film coating applied may vary 1 - 5 % (w/w) of core tablet. Preferred applied film coating is 3 % (w/w) of core tablet.

### Example 6 (Manufacturing Process of Example 5)_

Manufacturing Process of Example 5:
1. Lactose Monohydrate, Otilonium Bromide, Pregelatinized Starch and Crospovidone are mixed.
2. Magnesium Stearate is added and mixed.
3. Otilonium Tablets are compressed with suitable punches having average target weight of 90 mg/tablet.
4. Trimebutine Maleate, Lactose Monohydrate and ½ of Croscarmellose Sodium are mixed.
5. PVP K-30 is dissolved in water.
6. Powder mixture in D is granulated with Fluid Bed Granulator (Vector VFC-30X) and dried until moisture content is less than 3% (w/w).
7. Dry granules are mixed with remaining part of Croscarmellose Sodium and Avicel PH 102.
8. Magnesium Stearate is added, mixed and Trimebutine Granules ready for compression is prepared.
9. Using IMA S250 ZS/M Tablet Press Machine, which is capable of compressing tablet-in-tablet, Otilonium Tablets are compressed with Trimebutine Granules.
10. Average target weight of final core tablets is 500 mg/tablet.
11. Core tablets are optionally film coated. Opadry II 85F18422 White can be used as film coating agent.
12. 18% (w/w) dispersion of Opadry II 85F18422 White is prepared in water.
13. Core tablets are film coated with above dispersion using Vector VHC Hi-Coater film coating machine until desired film coating applied.

### Example 7 (Bilayer Tablet)

Otilonium Bromide + Trimebutine Maleate Double Layer Tablet:

**Table 4**

| | **mg/tablet** | **% (w/w)*** | **Function** |
|---|---|---|---|
| Otilonium Layer | | | |
| Otilonium Bromide | 40.00 | 8.00 | Drug Substance |
| Lactose Monohydrate | 18.00 | 3.60 | Diluent |
| Pregelatinized Starch | 35.00 | 7.00 | Dry Binder |
| Crospovidone | 6.00 | 1.20 | Disintegrant |
| Magnesium Stearate | 1.00 | 0.20 | Lubricant |
| Total Otilonium Layer Weight | 100.00 | | |

| Trimebutine Layer | | | |
|---|---|---|---|
| Trimebutine Maleate | 200.00 | 40.00 | Drug Substance |
| Lactose Monohydrate | 45.00 | 9.00 | Diluent |
| PVP K-30 | 22.00 | 4.40 | Binder |
| Croscarmellose Sodium | 25.00 | 5.00 | Disintegrant |
| Microcrystalline Cellulose (Avicel PH 101) | 105.00 | 21.00 | Diluent |
| Magnesium Stearate | 3.00 | 0.60 | Lubricant |
| Total Trimebutine Layer Weight | 400.00 | | |
| Total Core Tablet Weight | 500.00 | | |

* % values are calculated according to final core tablet weight.

Core tablets are optionally coated with dispersion of film coating material (HPMC or PVA based) either dispersed in water or water and alcohol mixture or organic solvent. Total film coating applied may vary 1 - 5 % (w/w) of core tablet. Preferred applied film coating is 3 % (w/w) of core tablet.

### Example 8 (Manufacturing Process of Example 7)

Manufacturing Process of Example 7:
1. Lactose Monohydrate, Otilonium Bromide, Pregelatinized Starch and Crospovidone are mixed.
2. Magnesium Stearate is added, mixed and Otilonium Layer ready for compression is prepared.
3. Trimebutine Maleate, Lactose Monohydrate, ½ of Croscarmellose Sodium and Avicel PH 101 are mixed.
4. PVP K-30 is dissolved in water+alcohol (70:30) mixture.
5. Powder mixture in D is granulated with high shear mixer (Collette UltimaGral).
6. Wet granules are transferred to fluid bed drier (Glatt) and dried with fluidized hot air until moisture content is less than 3% (w/w).
7. Remaining part of Croscarmellose Sodium is mixed with Dry Granules in Step F.
8. Magnesium Stearate is added, mixed and Trimebutine Layer ready for compression is prepared.
9. Double layer tablets are compressed from Otilonium Layer and Trimebutine Layer using Fette 102i Tablet Press Machine.
10. Average target weight of final core tablets is 500 mg/tablet.
11. Core tablets are optionally film coated. Opadry II 85F18422 White can be used as film coating agent.
12. 18% (w/w) dispersion of Opadry II 85F18422 White is prepared in water.
13. Core tablets are film coated with above dispersion using Vector VHC Hi-Coater film coating machine until desired film coating applied.

### Example 9 (Capsule)_

Otilonium Bromide + Trimebutine Maleate Capsule:

**Table 5**

| | **mg/capsule** | **% (w/w)** | **Function** |
|---|---|---|---|
| **Active Substances** | | | |
| Otilonium Bromide | 40.00 | 8.00 | Drug Substance |
| Trimebutine Maleate | 200.00 | 40.00 | Drug Substance |

| **Excipients** | | | |
|---|---|---|---|
| Lactose Monohydrate | 35.00 | 7.00 | Diluent |
| Pregelatinized Starch | 45.00 | 9.00 | Binder |
| Crospovidone | 40.00 | 8.00 | Disintegrant |
| Microcrystalline Cellulose (Avicel PH 102) | 137.00 | 27.40 | Diluent |
| Magnesium Stearate | 3.00 | 0.60 | Lubricant |
| Total Powder Weight in Capsule | 500.00 | 100.00 | |

### Example 10 (Manufacturing Process of Example 9)

Manufacturing Process of Example 9:
1. Lactose Monohydrate, Otilonium Bromide, Crospovidone, Pregelatinized Starch, Avicel PH 102 and Trimebutine Maleate are mixed.
2. Magnesium Stearate is added and mixed.
3. Powder mixture in Step C is filled into hard gelatin capsules having average target powder weight of 500 mg/capsule.

### Example 11 (Unit Formula)

Otilonium Bromide + Trimebutine Maleate Tablet Unit Formula:

**Table 6**

| | **mg/capsule** | **% (w/w)** | **Function** |
|---|---|---|---|
| **Active Substances** | | | |
| Otilonium Bromide | 40.00 | 8.00 | Drug Substance |
| Trimebutine Maleate | 200.00 | 40.00 | Drug Substance |

| **Excipients** | | | |
|---|---|---|---|
| Lactose Monohydrate | 35.00 | 7.00 | Diluent |
| Pregelatinized Starch | 25.00 | 5.00 | Binder |
| Crospovidone | 40.00 | 8.00 | Disintegrant |
| Microcrystalline Cellulose (Avicel PH 102) | 155.00 | 31.00 | Diluent |
| Magnesium Stearate | 5.00 | 1.00 | Lubricant |
| Total Powder Weight in Capsule | 500.00 | 100.00 | |

Core tablets are optionally coated with dispersion of film coating material (HPMC or PVA based) either dispersed in water or water and alcohol mixture or organic solvent. Total film coating applied may vary 1 - 5 % (w/w) of core tablet. Preferred applied film coating is 3 % (w/w) of core tablet.

### Example 12 (Manufacturing Process of Example 11 by Direct Compression)

Manufacturing Process of Example 11 by means of Direct Compression:
1. Lactose Monohydrate, Otilonium Bromide, Crospovidone, Pregelatinized Starch, Avicel PH 102 and Trimebutine Maleate are mixed.
2. Magnesium Stearate is added and mixed.
3. Powder mixture in Step C is compressed with suitable punches having average target tablet weight of 500 mg/tablet.
4. Core tablets are optionally film coated. Opadry II 85F18422 White can be used as film coating agent.
5. 18% (w/w) dispersion of Opadry II 85F18422 White is prepared in water.
6. Core tablets are film coated with above dispersion using Vector VHC Hi-Coater film coating machine until desired film coating applied.

### Example 13 (Unit Formula)

Otilonium Bromide + Trimebutine Maleate Tablet: Unit Formula:

**Table 7**

| | **mg/capsule** | **% (w/w)** | **Function** |
|---|---|---|---|
| **Active Substances** | | | |
| Otilonium Bromide | 40.00 | 13.33 | Drug Substance |
| Trimebutine Maleate | 200.00 | 66.67 | Drug Substance |

| **Excipients** | | | |
|---|---|---|---|
| Lactose Monohydrate | 10.00 | 3.33 | Diluent |
| Pregelatinized Starch | 36.50 | 12.17 | Dry Binder |
| Crospovidone | 12.00 | 4.00 | Disintegrant |
| Magnesium Stearate | 1.50 | 0.50 | Lubricant |
| Total Core Tablet Weight | 300.00 | 100.00 | |

Core tablets are optionally coated with dispersion of film coating material (HPMC or PVA based) either dispersed in water or water and alcohol mixture or organic solvent. Total film coating applied may vary 1 - 5 % (w/w) of core tablet. Preferred applied film coating is 3 % (w/w) of core tablet.

### Example 14 (Manufacturing Process of Example 13 by Dry Granulation)

Manufacturing Process of Example 13 by Dry Granulation:
1. Lactose Monohydrate, Otilonium Bromide, 2/3 of Crospovidone, Pregelatinized Starch and Trimebutine Maleate are mixed.
2. Powder mixture in Step A is dry granulated with roller compactor (Powtec) or slugged with suitable punches.
3. Compacted granules or slug tablets are crushed and sieved through 0.8 mm sieve.
4. Magnesium Stearate is added and mixed.
5. Powder mixture in Step D is compressed with suitable punches having average target tablet weight of 300 mg/tablet.
6. Core tablets are optionally film coated. Opadry II 85F18422 White can be used as film coating agent.
7. 18% (w/w) dispersion of Opadry II 85F18422 White is prepared in water.
8. Core tablets are film coated with above dispersion using Vector VHC Hi-Coater film coating machine until desired film coating applied.

### Example 15 (Tablet in Capsule : Unit Formula)

Otilonium Bromide + Trimebutine Maleate Tablet in Capsule:

**Table 8**

| | **mg/tablet** | **% (w/w)*** | **Function** |
|---|---|---|---|
| Otilonium Tablet | | | |
| Otilonium Bromide | 40.00 | 8.00 | Drug Substance |
| Lactose Monohydrate | 31.00 | 6.20 | Diluent |
| Pregelatinized Starch | 12.00 | 2.40 | Dry Binder |
| Crospovidone | 6.00 | 1.20 | Disintegrant |
| Magnesium Stearate | 1.00 | 0.20 | Lubricant |
| Total Otilonium Tablet Weight | 90.00 | | |

| Trimebutine Granule | | | |
|---|---|---|---|
| Trimebutine Maleate | 200.00 | 40.00 | Drug Substance |
| Lactose Monohydrate | 85.00 | 17.00 | Diluent |
| PVP K-30 | 32.00 | 6.40 | Binder |
| Croscarmellose Sodium | 40.00 | 8.00 | Disintegrant |
| Microcrystalline Cellulose (Avicel PH 102) | 50.00 | 10.00 | Diluent |
| Magnesium Stearate | 3.00 | 0.60 | Lubricant |
| Total Trimebutine Granule Weight | 410.00 | | |
| Total Weight Present in Capsule | 500.00 | | |

* % values are calculated according to final weight in capsule.

### Example 16 (Manufacturing Process of Example 15)_

Manufacturing Process of Example 15:
1. Lactose Monohydrate, Otilonium Bromide, Pregelatinized Starch and Crospovidone are mixed.
2. Magnesium Stearate is added and mixed.
3. Otilonium Tablets are compressed with suitable punches having average target weight of 90 mg/tablet.
4. Trimebutine Maleate, Lactose Monohydrate and ½ of Croscarmellose Sodium are mixed.
5. PVP K-30 is dissolved in water.
6. Powder mixture in D is granulated with Fluid Bed Granulator (Vector VFC-30X) and dried until moisture content is less than 3% (w/w).
7. Dry granules are mixed with remaining part of Croscarmellose Sodium and Avicel PH 102.
8. Magnesium Stearate is added, mixed and Trimebutine Granules ready for encapsulation is prepared.
9. Using Zanasi 40E Capsule Filling Machine, which is capable of filling both tablet and powder into capsule, Otilonium Tablets and Trimebutine Granules are filled into hard gelatin capsule.
10. Average target weight inside of capsules is 500 mg/capsule.

### Example 17 (Effervescent Tablet: Unit Formula)_

Otilonium Bromide + Trimebutine Maleate Effervescent Tablet:Unit Formula:

**Table 9**

| | **mg/tablet** | **% (w/w)** | **Function** |
|---|---|---|---|
| **Active Substances** | | | |
| Otilonium Bromide | 40.00 | 2.67 | Drug Substance |
| Trimebutine Maleate | 200.00 | 13.33 | Drug Substance |

| **Excipients** | | | |
|---|---|---|---|
| Sodium Bicarbonate | 690.00 | 46.00 | Effervescent Agent (Basic) |
| Citric Acid Monohydrate | 430.00 | 28.66 | Effervescent Agent (Acidic) |
| Sucralose | 25.00 | 1.67 | Sweetener |
| Sodium Saccharin | 35.00 | 2.33 | Sweetener |
| Maltodextrin | 40.00 | 2.67 | Diluent |
| Lemon Flavour | 30.00 | 2.00 | Flavouring Agent |
| Sodium Stearyl Fumarate | 10.00 | 0.67 | Lubricant |
| Total Tablet Weight | 1500.00 | 100.0 | |

### Example 18 (Manufacturing Process of Example 17)

Manufacturing Process of Example 17:
1. Trimebutine Maleate, Sodium Bicarbonate, Citric Acid Monohydrate are mixed.
2. Otilonium Bromide, Sucralose, Sodium Saccharin, Maltodextrin and Lemon Flavour are dissolved in water.
3. Powder mixture in Step A is granulated with Solution prepared in Step B utilizing fluid bed granulator. Fluid bed granulator may optionally have dehumidification system in order to reduce humidity of fluidized air to less than 40 % RH.
4. Granules are dried until moisture content is less than 1% (w/w).
5. Sodium Stearyl Fumarate is added to dry granules and mixed.
6. Effervescent tablets are compressed with suitable punches having average target weight 1500 mg/tablet.

### Example 19

Otilonium Bromide + Trimebutine Maleate Readily Dissolved Granules: Unit Formula:

**Table 10**

| | **mg/tablet** | **% (w/w)** | **Function** |
|---|---|---|---|
| **Active Substances** | | | |
| Otilonium Bromide | 40.00 | 6.67 | Drug Substance |
| Trimebutine Maleate | 200.00 | 33.33 | Drug Substance |

| **Excipients** | | | |
|---|---|---|---|
| Sucralose | 45.00 | 7.50 | Sweetener |
| Acesulfame Potassium | 20.00 | 3.33 | Sweetener |
| Maltodextrin | 280.00 | 46.67 | Diluent |
| Cherry Flavour | 15.00 | 2.50 | Flavouring Agent |
| Total Tablet Weight | 600.00 | 100.0 | |

### Example 20 (Manufacturing Process of Example 19)

Manufacturing Process of Example 19:
1. Trimebutine Maleate and Maltodextrin are mixed.
2. Otilonium Bromide, Sucralose, Acesulfame Potassium and Cherry Flavour are dissolved in water.
3. Powder mixture in Step A is granulated with Solution prepared in Step B utilizing fluid bed granulator. Fluid bed granulator may optionally have dehumidification system in order to reduce humidity of fluidized air to less than 50 % RH.
4. Granules are dried until moisture content is less than 1% (w/w).
5. Dried granules are filled into suitable packaging material, such as sachets, having average target powder weight of 600 mg/package.

### Example 21 (Readily Dispersible Granules)

Otilonium Bromide + Trimebutine Maleate Readily Dispersible Granules:Unit Formula:

**Table 11**

| | **mg/tablet** | **% (w/w)** | **Function** |
|---|---|---|---|
| **Active Substances** | | | |
| Otilonium Bromide | 40.00 | 6.67 | Drug Substance |
| Trimebutine Maleate | 200.00 | 33.33 | Drug Substance |

| Excipients | | | |
|---|---|---|---|
| Sucralose | 45.00 | 7.50 | Sweetener |
| Acesulfame Potassium | 20.00 | 3.33 | Sweetener |
| Carmellose Sodium | 90.00 | 15.00 | Suspending Agent |
| Mannitol | 190.00 | 31.67 | Diluent |
| Orange Flavour | 15.00 | 2.50 | Flavouring Agent |
| Total Tablet Weight | 600.00 | 100.0 | |

### Example 22 (Manufacturing Process of Example 21)

Manufacturing Process of Readily Dispersible Granules:
1. Trimebutine Maleate, Carmellose Sodium and Mannitol are mixed.
2. Otilonium Bromide, Sucralose, Acesulfame Potassium and Orange Flavour are dissolved in water.
3. Powder mixture in Step A is granulated with Solution prepared in Step B utilizing fluid bed granulator. Fluid bed granulator may optionally have dehumidification system in order to reduce humidity of fluidized air to less than 40 % RH.
4. Granules are dried until moisture content is less than 1% (w/w).
5. Dried granules are filled into suitable packaging material, such as sachets, having average target powder weight of 600 mg/package.

## Claims

1. A pharmaceutical composition or combination comprising; i) therapeutically effective amount of otilonium or pharmaceutically acceptable salt thereof; and ii) therapeutically effective amount of trimebutine or pharmaceutically acceptable salt thereof; and iii) a pharmaceutically acceptable carrier and/or diluent or mixtures thereof.

2. In pharmaceutical composition or combination, according to preceding claim, otilonium is present in an amount of from 10 mg to 150 mg, preferably in an amount of from 10 mg to 60 mg and most preferably 40 mg and trimebutine is present in an amount of from 10 mg to 500 mg, preferably in an amount of from 20 mg to 400 mg and most preferably 200 mg.

3. According to claim 2, a pharmaceutical composition or combination for preventing or treating of: **i)** Gastro intestinal symptoms due to chronic gastritis, **ii)** Gastro intestinal tract spasm, **iii)** irritable bowel syndrome and/or symptoms associated with the irritable bowel syndrome, **iv)** abdominal distention, abdominal pain, nausea, constipation, diarrhea, eructation, **v)** In postoperative paralytic ileus in order to accelerate the resumption of the intestinal transit following abdominal surgery

4. According to preceding claims, pharmaceutical composition or combination is prepared by a method; **i)** both active agents are separately granulated and then granulates are combined or, **ii)** one active agent is granulated and then granulate is combined with non granulated active agent or, **iii)** both active pharmaceutical ingredients are granulated together or, **iv)** one active agent is dissolved alone or with one or more excipients and then other active agent alone or with one or more excipients are coated with said solution, **v)** one active agent is dispersed alone or with one or more excipient and then other active agent alone or with one or more excipients are coated or adhered with said dispersion

5. According to claims of 1 to 2, pharmaceutical composition or combination is prepared by a method : **i)** otilonium and trimebutine are separately granulated and then they are separately compressed into mini tablets and filled into capsule altogether or, **ii)** tilonium and trimebutine are separately granulated and then they are separately compressed into tablet and filled into capsule or, iii) one of the active agents, otilonium or trimebutine, is granulated and compressed into tablet or mini-tablets and then combined with granules of other active agent, otilonium or trimebutine, into capsule or **iv)** otilonium and trimebutine are separately granulated and then directly filled into capsule without tabletting or, **v)** one of the active agents, otilonium or trimebutine, is prepared through direct compression into tablet or mini-tablets and then combined with granules of other active agent, otilonium or trimebutine, into capsule.

6. According to claims of 1 to 2, pharmaceutical composition or combination is in the form of tablet-in-tablet which comprises: **a)** a core tablet comprising: trimebutine or pharmaceutically acceptable salts thereof and a pharmaceutically acceptable carrier or carriers and, **b)** a compressed outer tablet layer comprising: otilonium or pharmaceutically acceptable salts thereof and a pharmaceutically acceptable carrier or carriers **or:a)** a core tablet comprising: otilonium or pharmaceutically acceptable salts thereof and a pharmaceutically acceptable carrier or carriers and, **b)** a compressed outer tablet layer comprising: trimebutine or pharmaceutically acceptable salts thereof and a pharmaceutically acceptable carrier or carriers.

7. According to claims of 1 to 2, pharmaceutical composition or combination is in the form of bilayer tablet which comprises; **a)** a first layer containing trimebutine or pharmaceutically acceptable salts thereof and a pharmaceutically acceptable carrier or carriers and, **b)** a second layer containing otilonium or pharmaceutically acceptable salts thereof and a pharmaceutically acceptable carrier or carriers **or: a)** a first layer containing otilonium or pharmaceutically acceptable salts thereof and a pharmaceutically acceptable carrier or carriers and **b)** a second layer containing trimebutine or pharmaceutically acceptable salts thereof and a pharmaceutically acceptable carrier or carriers.

8. According to claims of 1 to 2, pharmaceutical composition or combination is in the form of inlay tablet which comprises **a)** an inner tablet comprising otilonium or pharmaceutically acceptable salts thereof and a pharmaceutically acceptable carrier or carriers and **b)** an outer tablet comprising trimebutine or pharmaceutically acceptable salts thereof and a pharmaceutically acceptable carrier or carriers **or:a)** an inner tablet comprising trimebutine or pharmaceutically acceptable salts thereof and a pharmaceutically acceptable carrier or carriers and **b)** an outer tablet comprising otilonium or pharmaceutically acceptable salts thereof and a pharmaceutically acceptable carrier or carriers wherein inner tablet is encompassed by an outer tablet wherein at least a portion of one surface of the inner tablet is not surrounded by outer tablet.

9. Pharmaceutical composition or combination , according to claims of 1 to 2, is in the form of capsule in capsule which consists of **a)** an external capsule comprising trimebutine or pharmaceutically acceptable salts thereof and a pharmaceutically acceptable carrier or carriers and **b)** internal capsule (inner capsule) comprising otilonium or pharmaceutically acceptable salts thereof and a pharmaceutically acceptable carrier or carriers **or:a)** an external capsule comprising otilonium or pharmaceutically acceptable salts thereof and a pharmaceutically acceptable carrier or carriers and **b)** internal capsule (inner capsule) comprising trimebutine or pharmaceutically acceptable salts thereof and a pharmaceutically acceptable carrier or carriers wherein smaller size capsule is encapsulated into a larger capsule.

10. Pharmaceutical composition or combination, according to claims of 1 to 2, comprises spray dried granules of trimebutine or pharmaceutically acceptable salts thereof and spray dried granules of otilonium or pharmaceutically acceptable salts thereof and preferably an excipient or mixtures of excipients.

11. Pharmaceutical composition or combination, according to claims of 1 to 2, is prepared by dry granulation or direct compression or wet granulation or melt granulation or mixtures thereof.

12. Pharmaceutical composition or combination, according to claims of 1 to 2, is in the form of tablet in capsule which comprises trimebutine or pharmaceutically acceptable salts thereof and otilonium or pharmaceutically acceptable salts thereof and preferably an excipient or mixtures of excipients.

13. Pharmaceutical composition or combination, according to claims of 1 to 2, is in the form of effervescent tablet or effervescent granule or effervescent pellet or effervescent dry powder which comprises trimebutine or pharmaceutically acceptable salts thereof and otilonium or pharmaceutically acceptable salts thereof and preferably an excipient or mixtures of excipients.

14. According to claims 1 to 2, pharmaceutical combination is in the form of kit which comprises **i)** therapeutically effective amount otilonium and optionally an acceptable carrier or diluent in a unit dosage form and **ii)** therapeutically effective amount of trimebutine and an acceptable carrier or diluent in a unit dosage form and **iii)** a container.

15. Pharmaceutical composition or combination, according to claims of 1 to 2, is readily dispersible or readily dissolved granules.
